Europäisches Patentamt

(19) European Patent Office

Office européen des brevets

(11) Numéro de publication : **0 314 576 B1**

(12) # FASCICULE DE BREVET EUROPEEN

(45) Date de publication du fascicule du brevet :
02.05.91 Bulletin 91/18

(21) Numéro de dépôt : **88402713.7**

(22) Date de dépôt : **27.10.88**

(51) Int. Cl.⁵ : **C07K 5/10**, C07K 7/06, C12P 21/02, A61K 37/02

(54) Tétra- et pentapeptides contenant la séquence lysyl-arginyl- aspartyle et leurs applications en tant que médicaments, notamment antithrombotiques.

(30) Priorité : 30.10.87 FR 8715061

(43) Date de publication de la demande :
03.05.89 Bulletin 89/18

(45) Mention de la délivrance du brevet :
02.05.91 Bulletin 91/18

(84) Etats contractants désignés :
AT BE CH DE ES FR GB GR IT LI LU NL SE

(56) Documents cités :
EP-A- 0 067 425
EP-A- 0 220 957
CHEMICAL ABSTRACTS, vol. 109, 1988, page 450, résumé no. 126475w, Columbus, Ohio, US; S. RAHA et al.: "KRDS - a tetrapeptide derived from lactotransferrin - inhibits binding of monoclonal antibody against glycoprotein IIb-IIa on ADP-stimulated platelets and mega-karyocytes", & BLOOD 1988, 72(1), 172-8
EUROPEAN JOURNAL OF BIOCHEMISTRY, vol. 145, no. 3, 17 décembre 1984, pages 659-676, Springer International; M.-H. METZ-BOUTIGUE et al.: "Human lactotransferrin: amino acid sequence and structural compari-sons with other transferrins"
CHEMICAL ABSTRACTS, vol. 103, 1985, page 627, résumé no. 212136u, Columbus, Ohio, US; D.M. HAVERSTICK et al.: "Inhibition of plate-let adhesion to fibronectin, fibrinogen, and von Willebrand factor substrates by a synthe-tic tetrapeptide derived from the cell-binding domain of fibronectin", & BLOOD 1985, 66(4), 946-52

(73) Titulaire : **INSTITUT DES VAISSEAUX ET DU SANG**
**Hôpital Lariboisière 8, rue Guy Patin**
**F-75010 Paris (FR)**
Titulaire : **CENTRE NATIONAL DE LA RECHERCHE SCIENTIFIQUE**
**15, quai Anatole France**
**F-75007 Paris (FR)**

(72) Inventeur : **Jolles, Pierre**
**2, Rue Jean-François Gerbillon**
**F-75006 Paris (FR)**
Inventeur : **Fiat, Anne-Marie**
**67 bis Avenue des Pages**
**F-78110 Le Vesinet (FR)**
Inventeur : **Soria, Claudine**
**66, Rue de Paris**
**F-95150 Taverny (FR)**
Inventeur : **Levy-Toledano, Sylviane**
**16, Rue des Sablons**
**F-75116 Paris (FR)**
Inventeur : **Raha, Sanghamitra,Indian Institute of**
**Chemical Biology 4, raja SC Mulliek Road**
**Calcutta 700 032 (IN)**
Inventeur : **Mazoyer, Elizabeth**
**85, Rue Cardinal Lemoine**
**F-75005 Paris (FR)**
Inventeur : **Drouet, Ludovic**
**14, Avenue des Cottages**
**F-92340 Bourg-la-Reine (FR)**

(74) Mandataire : **Ayache, Monique et al**
**Cabinet Ayache 8 rue Beaumarchais**
**F-92500 Rueil-Malmaison (FR)**

## Description

L'invention a pour objet de nouveaux tétra- et pentapeptides à activité antithrombotique.

Plus précisément, l'invention a pour objet des tétra- et pentapeptides contenant la séquence Lys-Arg-Asp et leurs applications thérapeutiques, notamment en tant qu'agents antithrombotiques.

Il est entendu que ci-dessus, ainsi que dans la description et les revendications qui suivent, tous les peptides sont représentés avec le résidu amino-terminal à gauche et sont tous sous la forme L.

Un grand nombre de faits, accumulés ces dernières années, tendent à indiquer que le récepteur du fibrinogène est situé sur un complexe de deux glycoprotéines plaquettaires GP IIb et GP IIIa, sur la membrane plasmatique des plaquettes. La liaison du fibrinogène au récepteur plaquettaire nécessite l'activation des plaquettes par des agonistes tels que l'ADP, la thrombine etc., ce qui semble avoir pour effet d'exposer les sites de liaison du fibrinogène (voir par exemple W.M. Isenberg et coll. dans Blood, novembre 1986, n°1150).

Un tétrapeptide synthétique, Arg-Gly-Asp-Ser contenant la séquence Arg-Gly-Asp commune à des séquences de la chaîne α du fibrinogène, du Facteur de von Willebrand (vWF) et de la fibronectine et une séquence (400-411) de la chaîne γ du fibrinogène sont connus en tant qu'inhibiteurs de la liaison du fibrinogène. Pierre JOLLES et coll. (Eur. J. Biochem. 158, 379-382 (1986)) ont noté des similitides entre des séquences peptidiques de la chaîne γ du fibrinogène (400-411) et de la caséine K (106-116) provenant de la caséine de lait de vache dans leur aptitude à inhiber l'agrégation plaquettaire et la liaison du fibrinogène. Toutefois, les effets de ces séquences peptidiques inhibitrices sur la liaison d'anticorps monoclonaux dirigés contre le complexe glycoprotéique GP IIb-IIIa n'ont pas été étudiés.

Ceci étant, les demandeurs, poursuivant les investigations entreprises dans ce domaine, ont eu l'idée de synthétiser et d'étudier l'activité pharmacologique d'une famille de tétra- et pentapeptides contenant la séquence Lys-Arg-Asp, et notamment le tétrapeptide Lys-Arg-Asp-Ser qui correspond à la séquence 39 → 42 de la lactotransferrine humaine dont la séquence d'amino-acides complète établie dans le Laboratoire des Protéines, dirigé par le Professeur Pierre JOLLES, à l'Université de Paris V, est décrite par Marie-Hélène METZ-BOUTIGUE et coll. dans Eur. J. Biochem. 145, 659-676 (1984). Le document EP-A-0 067 425 décrit, entre autres, un tétrapeptide synthétique Lys-Arg-Asp-Val ayant une activité régulatrice sur le système immunitaire mais ne suggère aucunement qu'il pourrait avoir une activité antithrombotique.

Selon l'un de ses aspects, l'invention a pour objet un peptide répondant à la formule générale :

$$\text{Z-Lys-Arg-Asp-X-Y} \quad \text{(I)}$$

dans laquelle :

– X représente le résidu de l'un des 20 amino-acides usuels sous forme L,

– Y représente hydroxyle ou le résidu C-terminal de l'un des 20 amino-acides usuels sous forme L,

– Z représente acétyle, hydrogène ou le résidu N-terminal de l'un des 20 amino-acides usuels sous Forme L, éventuellement N acétylé, étant entendu que :

– lorsque Y représente le résidu C-terminal de l'un des 20 amino-acides usuels sous forme L, Z représente acétyle ou hydrogène et

– X peut dans tous les cas être différent de Y et de Z.

Il est rappelé que les "20 amino-acides usuels" sont :

l'alanine (Ala) ; l'arginine (Arg) ; l'asparagine (Asn) ; l'acide aspartique (Asp) ; la cystéine (Cys) ; la glutamine (Gln) ; l'acide glutamique (Glu) ; la glycine (Gly) ; l'histidine (His) ; l'isoleucine (Ile) ; la leucine (Leu) ; la lysine (Lys) ; la méthionine (Met) ; la phénylalanine (Phe) ; la proline (Pro) ; la sérine (Ser) ; la thréonine (Thr) ; le tryptophane (Trp) ; la tyrosine (Tyr) ; et la valine (Val).

Les peptides de formule générale I peuvent être obtenus par synthèse peptidique classique ou, plus simplement, en ayant recours à un synthétiseur de pep ies.

Comme le montre l'exposé figurant dans la part expérimentale qui suit, les peptides selon l'invention non seulement sont capables d'inhiber l'agrégation pla ettaire et la liaison du fibrinogène sur des plaquettes stimulées, mais sont en outre capables d'inhiber la liaison d'anticorps monoclonaux dirigés contre le complexe GP IIb-GP IIIa sur des plaquettes stimulées.

Par ailleurs, les peptides selon l'invention, et notamment ceux répondant à la formule générale :

$$\text{Lys-Arg-Asp-X,} \quad \text{(II)}$$

agissent synergiquement avec d'autres peptides à activité antithrombotique connus, notamment avec le pep-

EP 0 314 576 B1

tide Arg-Gly-Asp-Ser.

De plus, les mégacaryocytes (MK), tout comme les plaquettes, peuvent être stimulés par l'ADP pour exposer les sites de liaison à ces peptides et cette aptitude est limitée à un groupe de mégacaryocytes qui sont de façon prédominante matures, si l'on en juge au moyen de critères morphologiques.

Compte tenu de ces propriétés, les peptides de formule générale I selon l'invention peuvent être utilisés en thérapeutique, notamment dans la prévention et le traitement des thromboses, seuls ou en mélange entre eux et/ou avec d'autres peptides à activité antithrombotique connus.

Ils peuvent être alors utilisés par voies intraveineuse, intramusculaire, sous-cutanée, intra-nasale, éventuellement orale ou rectale, soit encore sous forme de liposomes.

Les doses administrées seront en général de 0,05 à 5 mg/kg de poids corporel, de 3 à 5 fois par jour.

L'exposé qui suit est destiné à mieux expliquer l'invention.

## I. Exemples de synthèse et de purification :

### A. Synthèse, purification et analyse du peptide Lys-Arg-Asp-Ser (peptide 1)

Ce peptide a été synthétisé à l'aide du synthétiseur de peptides de la société Applied Biosystems commercialisé sous la référence 430A.

Il a ensuite été purifié par chromatographie liquide haute performance (HPLC) dans les conditions suivantes :

Colonne : de 4,5 × 300 mm, silice greffée, phase inverse, porosité 100 Å, diamètre des particules 5 μm, commercialisée sous la dénomination Nucleosil® C 18 par la société MACHEREY NAGEL.

Eluant : eau/acétonitrile/acide trifluoracétique dans les proportions volumétriques 970 : 30 : 1.

Débit : 1 ml/min.

Lecture à 215 nm.

Le peptide a été élué après 4,76 minutes. Il a ensuite été lyophilisé.

### Analyse

Après hydrolyse totale par HCl 6N + mercapto-2 éthanol au 1/2000è, pendant 18 heures à 110°C sous vide, on a trouvé que la composition en acides aminés (résidus/mole) était la suivante :

Asp : 1,04
Ser : 0,91
Lys : 1,02
Arg : 0,92

en utilisant un appareil Biotronik®, modèle LC 6000 commercialisé par la société SEMSA BIOTRONIK.

La séquence du peptide a été établie à l'aide du séquenceur de la société Applied Biosystems, référence 470A.

Les phénylthiohydantoïnes (PTH) des acides aminés ont été identifiées par HPLC avec le chromatographe de la société Applied Biosystems référence 120A.

La séquence trouvée est la suivante :

$$\underset{\longrightarrow}{Lys} \quad \underset{\longrightarrow}{Arg} \quad \underset{\longrightarrow}{Asp} \quad \underset{\longrightarrow}{Ser} \; *$$

$*\underset{\longrightarrow}{}$ : amino acide déterminé par dégradation automatique d'Edman.

Aucune trace d'impureté n'a pu être décelée. Ce peptide est donc pur à 99% au moins.

### Caractéristiques physiques :

Ce peptide est hydrophile et a une très bonne solubilité dans l'eau.

3

## B. Synthèse, purification et analyse d'autres peptides de formule générale I.

Les autres peptides de formule générale I peuvent être synthétisés, purifiés et analysés de manière analogue à ce qui a été décrit pour le peptide Lys-Arg-Asp-Ser.

On donnera ici, à titre d'exemples, les résultats d'analyse obtenus pour les peptides :

Lys-Art-Asp-Tyr (X = Tyr, Y = OH et Z = H) (peptide 2),
Tyr-Lys-Arg-Asp-Ser (X = Ser, Y = OH et Z = Tyr) (peptide 3),
Ac-Lys-Arg-Asp-Ser-Lys (X = Ser, Y = Lys et Z = Ac) (peptide 4),
Lys-Arg-Asp-Ser-Tyr (X = Ser, Y = Tyr et Z= H) (peptide 5) et
Lys-Arg-Asp-Arg (X = Arg, Y = OH et Z = H) (peptide 6).

Les compositions en acides aminés trouvées sont récapitulées dans le tableau qui suit :

| N° du peptide | Résidu d'acide aminé | | | | |
|---|---|---|---|---|---|
| | Asp | Ser | Tyr | Lys | Arg |
| 2 | 0,99 | - | 0,99 | 0,99 | 1,02 |
| 3 | 1,00 | 1,00 | 0,99 | 1,00 | 1,05 |
| 4 | 0,89 | 0,86 | - | 2,09 | 1,02 |
| 5 | 0,96 | 0,81 | 1,02 | 1,09 | 1,12 |
| 6 | 1,00 | - | - | 1,05 | 1,99 |

Les séquences trouvées par dégradation automatique d'Edman sont les suivantes :

Peptide 2    Lys →   Arg →   Asp →   Tyr →

Peptide 3    Tyr →   Lys →   Arg →   Asp →   Ser →

Peptide 4    rien "en direct"

Peptide 5    Lys →   Arg →   Asp →   Ser →   Tyr →

Peptide 6    Lys →   Arg →   Asp →   Arg →

Après hydrolyse trypsique, la séquence trouvée pour le peptide 4 est :

$$Arg \longrightarrow$$
$$Asp \longrightarrow \quad Ser \longrightarrow \quad Lys \longrightarrow$$

II. Etude pharmacologique :

A. Etudes in vitro

1. Etude comparative avec des peptides connus

On a effectué une étude comparative du tétrapeptide 1, Lys-Arg-Asp-Ser, d'une part et de deux peptides connus comme présentant une activité inhibitrice vis-à-vis de la liaison du fibrinogène, à savoir le peptide Arg-Gly-Asp-Ser signalé plus haut et le peptide VL 10 qui représente les dix acides aminés terminaux de la portion C-terminale de la chaîne γ du fibrinogène, d'autre part en ce qui concerne certaines activités pharmacologiques.

Plus précisément, l'étude a porté sur l'agrégation plaquettaire, la liaison du fibrinogène induite par l'ADP et la liaison d'anticorps monoclonaux dirigés contre les glycoprotéines GP IIb-IIIa sur les plaquettes humaines. La liaison d'un anticorps monoclonal contre le complexe glycoprotéinique GP IIb-IIIa sur les mégacaryocytes (MK) humains a été également étudiée après traitement au moyen des peptides inhibiteurs des plaquettes. La liaison des anticorps monoclonaux aux plaquettes et aux mégacaryocytes a été visualisée et jugée qualitativement par une méthode indirecte à l'immunoperoxydase [Beckstead et coll., Blood, 67, 285 (1986)].

Une concentration 1mM de peptide 1 inhibe à la fois l'agrégation plaquettaire et la liaison du fibrinogène induite par l'ADP, pratiquement de façon complète. Parmi les trois peptides étudiés, seul le peptide 1 selon l'invention (à une concentration 450 ou surtout 900 μM) inhibe la liaison d'anticorps monoclonaux dirigés contre le complexe GP IIb-IIIa, à savoir, dans le cadre de cette étude, les anticorps monoclonaux AP2 et P2 qui sont l'un et l'autre dirigés contre un épitope de la membrane plaquettaire constitué par le complexe des glycoprotéines IIb et IIIa.

Il est à ce propos précisé que l'anticorps AP2 est un anticorps monoclonal de souris contre le complexe glycoprotéinique plaquettaire IIb-IIIa humain [Pidard et coll., J. Biol. Chem., 258, 12582 (1983)] et l'anticorps P2 est un anticorps monoclonal de souris contre le complexe glycoprotéinique IIb-IIIa humain, préparé et commercialisé par la société IMMUNOTECH de Marseille (France).

Cette propriété du peptide 1 selon l'invention n'a été mise en évidence que sur les plaquettes stimulées par l'ADP et non sur les plaquettes non stimulées. Bien que la liaison tant de l'AP2 que du P2 soit inhibée par le peptide 1, la liaison du P2 est considérablement plus affectée que celle de l'AP2.

La liaison d'anticorps monoclonaux fabriqués à SUZHOU en République Populaire de Chine, à savoir l'anticorps monoclonal de souris SZ2 dirigé contre la glycoprotéine humaine plaquettaire Ib [Ruan et coll. Blood, 69, 570, (1987)], l'anticorps monoclonal SZ21 dirigé contre la glycoprotéine plaquettaire humaine IIIa et l'anticorps monoclonal de souris SZ22 dirigé contre la glycoprotéine humaine plaquettaire IIb [Ruan et coll., Thromb. Haemost. 58, 243A, (1987)] n'est pas inhibée par le peptide 1.

La liaison de l'AP2 radio-iodé sur des plaquettes stimulées par l'ADP est réduite de 30% par un prétraitement par le peptide 1, par comparaison aux plaquettes qui sont incubées avec du peptide 1 en l'absence de stimulation par l'ADP.

Le peptide 1 inhibe également la liaison du P2 sur une partie des mégacaryocytes, après stimulation par l'ADP. L'inhibition de la liaison du P2 sur les MK nécessite une concentration plus élevée en ADP (15 ou 20 μM) que dans le cas des plaquettes (5 μM), ce qui est le reflet d'une réponse inférieure des MK à l'ADP. Seul un faible pourcentage de la population totale en MK (21%) présente un inhibition de la liaison du P2. Le pourcentage de MK présentant une inhibition est également, parmi les MK matures, significativement supérieur (P < 0,001) à celui observé parmi les MK en période de maturation.

Ces données démontrent que le peptide 1 selon l'invention est capable d'inhiber non seulement l'agrégation plaquettaire et la liaison du fibrinogène sur des plaquettes stimulées, mais également la liaison d'un anticorps monoclonal dirigé contre le complexe GP IIb-IIIa sur les mégacaryocytes stimulés.

De plus, les MK, tout comme les plaquettes, peuvent être stimulés par l'ADP pour exposer les sites de liaison au peptide 1 et cette aptitude est limitée à une portion des MK qui sont matures de façon prédominante, si l'on en juge par les critères morphologiques.

## 2. Action spécifique in vitro sur l'activation plaquettaire et la phosphorylation indépendante.

Cette étude a également été effectuée sur le peptide Lys-Arg-Asp-Ser (peptide 1).

Afin de comprendre son mécanisme d'action, on a recherché s'il interférait avec le mécanisme de transduction du signal membranaire. En effet, il est établi que lorsqu'un agoniste tel que la thrombine se lie à la membrane plaquettaire, il active une phospholipase, la phospholipase C (PLC) qui hydrolyse le phosphatidylinositol 4,5 bis-phosphate ($PIP_2$) constitutif de la membrane. Cela donne naissance à deux très importants messagers: le diacylglycérol (DAG) et l'inositol trisphosphate ($IP_3$).

Par ailleurs, le DAG donne naissane à l'acide phosphatidique (PA), mais surtout, active la protéine kinase C qui phosphoryle une protéine de poids moléculaire 43000 (P43).

La protéine phosphorylée P43 a un rôle dans la réaction de libération.

Le peptide 1 inhibe l'agrégation à la thrombine. L'inhibition de l'agrégation à la thrombine s'accompagne d'une inhibition de la libération de sérotonine du même ordre de grandeur : 50 à 60%, avec une concentration 1000 µM de peptide 1.

Toutefois, le peptide 1, d'une part, n'affecte pas l'activité de la PLC puisque le $PIP_2$ est hydrolysé et le PA synthétisé et, d'autre part, ne modifie pas la phosphorylation de la P43 qui survient en présence de thrombine.

L'originalité de ce peptide, et des peptides selon l'invention en général, est donc d'inhiber la réaction de libération plaquettaire tout en maintenant une activité de la PLC ainsi qu'une phosphorylation de la P43.

## 3. Activité inhibitrice comparée de différents peptides selon l'invention sur l'agrégation plaquettaire à l'ADP

Cette activité en été mesurée in vitro avec des concentrations 1000 µM de chacun des peptides concernés. Les résultats obtenus sont rassemblés dans le tableau suivant.

| Peptide (n°) | Inhibition de l'agrégation plaquettaire en % |
|---|---|
| Lys-Arg-Asp-Ser (1) | 90 |
| Lys-Arg-Asp-Tyr (2) | 30 |
| Tyr-Lys-Arg-Asp-Ser (3) | 55 |
| Ac-Lys-Arg-Asp-Ser-Lys (4) | 67 |
| Lys-Arg-Asp-Ser-Tyr (5) | 60 |

## B. Etudes in vivo

### 1. Activité antithrombotique

Les travaux de thrombose expérimentale conduits dans deux espèces animales différentes (le rat et le cobaye) ont montré que les peptides selon l'invention, et en particulier les peptides répondant à la formule générale Lys-Arg-Asp-X (II), à savoir notamment le peptide Lys-Arg-Asp-Ser (peptide 1) et le peptide Lys-Arg-Asp-Arg (peptide 6), ont une activité antithrombotique sur un thrombus intravasculaire induit par une lésion spécifique de la paroi artérielle. Le type de thrombus qui est ainsi induit prend en compte la participation des plaquettes sanguines contre lesquelles le peptide exerce son activité mais aussi la réactivité de la paroi vasculaire lésée.

Cette activité antithrombotique in vivo a été démontrée :
– après injection du peptide concerné par voie intraveineuse directe ;
– pour des concentrations aussi basses que 0,5 mg/kg de poids d'animal d'expérience.

Cet effet antithrombotique dure plus de 80 minutes après l'injection. Il s'épuise pour les trop fortes concen-

6

trations (supérieures à 5 mg/kg de poids d'animal).

2. Activité synergique avec d'autres peptides antithrombotiques

Dans le même système de thrombose expérimentale, lorsqu'on utilise un peptide selon l'invention, notamment un peptide répondant à la formule Lys-Arg-Asp-X (II) et un peptide à activité antithrombotique connu tel que notamment le peptide Arg-Gly-Asp-Ser, à des concentrations très faibles, on observe une très forte inhibition durable de la formation de thrombi, alors que des doses doubles de chacun des peptides en question, utilisés séparément, ne produisent aucun effet ou seulement un effet modeste de très courte durée.

Ainsi, l'injection rapide simultanée de 250 µg/kg de peptide Arg-Gly-Asp-Ser et 125 µg/kg de peptide Lys-Arg-Asp-Ser chez le rat inhibe à 70% la formation de thrombi pendant une période allant jusqu'à 70 minutes.

En revanche, l'injection chez le même animal de 250 µg/kg de peptide selon l'invention (Lys-Arg-Asp-Ser) ne donne aucune inhibition, tandis que l'injection de 500 µg/kg de peptide Arg-Gly-Asp-Ser ne donne qu'une inhibition très modeste quine dure que quelques minutes.

En conclusion, les résultats obtenus tant in vitro qu'in vivo montrent que les peptides selon l'invention, et en particulier les peptides de formule générale Lys-Arg-Asp-X (II), en agissant probablement sur les plaquettes sanguines, inhibent la possibilité de ces cellules d'interagir normalement et constituent ainsi des molécules antithrombotiques.

**Revendications**

1. Peptide répondant à la formule générale Ia :

$$Z\text{-Lys-Arg-Asp-X-Y} \quad \text{(IA)}$$

dans laquelle :
- X représente le résidu de l'un des 20 amino-acides usuels sous forme L,
- Y représente hydroxyle ou le résidu C-terminal de l'un des 20 amino-acides usuels sous forme L,
- Z représente acétyle, hydrogène ou le résidu N-terminal de l'un des 20 amino-acides usuels sous forme L, éventuellement N-acétylé, étant entendu que :
- lorsque Y représente le résidu C-terminal de l'un des 20 aminoacides usuels sous forme L, Z représente acétyle ou hydrogène,
- X peut dans tous les cas être différent de Y et de Z, et
- lorsque Y est hydroxyle et Z est hydrogène, X n'est pas le résidu C-terminal de la valine.

2. Peptide selon la revendication 1, caractérisé en ce qu'il répond à la formule générale IIa :

$$\text{Lys-Arg-Asp-X} \quad \text{(IIA)}$$

dans laquelle X est défini comme à la revendication 1, étant entendu que X ne peut représenter le résidu C-terminal de la valine.

3. Le peptide Lys-Arg-Asp-Ser.

4. Peptide choisi dans le groupe constitué par :
- Lys-Arg-Asp-Tyr ;
- Tyr-Lys-Arg-Asp-Ser ;
- Ac-Lys-Arg-Asp-Ser-Lys ;
- Lys-Arg-Asp-Ser-Tyr ; et
- Lys-Arg-Asp-Arg.

5. Médicament, contenant en tant que principe actif, au moins un peptide selon l'une des revendications 1 à 4.

6. Médicament, contenant en tant que principe actif, au moins un peptide répondant à la formule générale I :

$$Z\text{-Lys-Arg-Asp-X-Y} \quad \text{(I)}$$

7

dans laquelle :
- X représente le résidu de l'un des 20 amino-acides usuels sous forme L,
- Y représente hydroxyle ou le résidu C-terminal de l'un des 20 amino-acides usuels sous forme L,
- Z représente acétyle, hydrogène ou le résidu N-terminal de l'un des 20 amino-acides usuels sous forme L, éventuellement N-acétylé, étant entendu que :
- lorsque Y représente le résidu C-terminal de l'un des 20 aminoacides usuels sous forme L, Z représente acétyle ou hydrogène et
- X peut dans tous les cas être différent de Y et de Z, notamment à la formule générale II :

$$Lys\text{-}Arg\text{-}Asp\text{-}X \quad (II)$$

dans laquelle X a la même signification que dans la formule générale I, conjointement à au moins un autre composé à activité antithrombotique.

7. Médicament, contenant en tant que principe actif au moins un peptide selon la revendication 3 ou 4, conjointement à au moins un autre composé à activité antithrombotique.

8. Médicament contenant en tant que principe actif au moins un peptide répondant à la formule générale I ou à la formule générale II, telles que définies à la revendication 6, conjointement au peptide Arg-Gly-Asp-Ser.

9. Médicament contenant en tant que principe actif au moins un peptide selon la revendication 3 ou 4, conjointement au peptide Arg-Gly-Asp-Ser.

10. Procédé pour la fabrication d'un médicament pour la prévention et/ou le traitement des thromboses, caractérisé en ce qu'il comprend l'incorporation dans une forme pharmaceutique convenable, éventuellement en présence d'un autre composé à activité antithrombotique, d'au moins un peptide répondant à la formule générale I ou à la formule générale II, telles que définies à la revendication 6.

## Ansprüche

1. Peptid der allgemeinen Formel Ia :

$$Z\text{-}Lys\text{-}Arg\text{-}Asp\text{-}X\text{-}Y, \quad (IA)$$

worin
- X als Rest für eine der 20 gewöhnlich vorkommenden L-Aminosäuren steht,
- Y eine Hydroxylgruppe oder der C-terminale Rest einer der 20 gewöhnlich vorkommenden L-Amininosäuren ist und
- Z einen Acetylrest, Wasserstoff oder den N-terminalen Rest einer der 20 gewöhnlich vorkommenden, möglicherweise N-acetylierten L-Aminosäuren darstellt, mit der Maßgabe, daß
- wenn Y der C-terminale Rest einer der 20 gewöhnlich vorkommenden L-Aminosäuren ist, Z ein Acetylrest oder Wasserstoff ist,
- X in allen Fällen von Y und Z verschieden sein kann und
- wenn Y eine Hydroxylgruppe ist und Z ein Wasserstoff ist, X ist nicht der C-terminale Rest von Valin.

2. Peptid nach Anspruch 1, dadurch gekennzeichnet, daß es die allgemeine Formel IIa

$$Lys\text{-}Arg\text{-}Asp\text{-}X \quad (IIA)$$

aufweist, worin X einen Rest wie in Anspruch 1 definiert darstellt, mit der Maßgabe, daß X nicht der C-terminale Rest von Valin sein kann.

3. Das Peptid mit der Sequenz Lys-Arg-Asp-Ser.

4. Peptid aus der Gruppe mit den folgenden Sequenzen :
- Lys-Arg-Asp-Tyr,
- Tyr-Lys-Arg-Asp-Ser,
- Ac-Lys-Arg-Asp-Ser-Lys,
- Lys-Arg-Asp-Ser-Tyr und
- Lys-Arg-Asp-Arg.

5. Medikament mit mindestens einem Peptid nach den Ansprüchen 1-4 als aktivem Bestandteil.

6. Medikament mit mindestens einem Peptid der allgemeinen Formel I :

$$Z\text{-}Lys\text{-}Arg\text{-}Asp\text{-}X\text{-}Y \quad \text{(I)}$$

als aktivem Bestandteil, worin
 – X der Rest für eine der 20 gewöhnlich vorkommenden L-Aminosäuren,
 – Y eine Hyydroxylgruppe oder der C-terminale Rest einer der 20 gewöhnlich vorkommenden L-Aminosäuren und
 – Z eine Acetylgruppe, Wasserstoff oder der N-terminale Rest einer der 20 gewöhnlich vorkommenden, möglicherweise N-acetylierten L-Aminosäuren sind, mit der Maßgabe, daß
 – wenn Y der C-terminale Rest einer der 20 gewöhnlich vorkommenden L-Aminosäuren ist, Z ein Acetylrest oder Wasserstoff ist und
 – X in allen Fällen verschieden von Y und Z sein kann,
vorzugsweise mit der allgemeinen Formel II :

$$Lys\text{-}Arg\text{-}Asp\text{-}X, \quad \text{(II)}$$

worin X die gleiche Bedeutung wie in der allgemeinen Formel I hat, gemeinsam mit mindestens einer anderen Verbindung mit antithrombotischer Aktivität.

7. Medikament mit mindestens einem Peptid nach Anspruch 3 oder 4 als aktivem Wirkstoff zusammen mit mindestens einer anderen Verbindung mit antithrombotischer Aktivität.

8. Medikament mit mindestens einem Peptid der wie in Anspruch 6 definierten allgemeinen Formel I oder II als aktivem Wirkstoff zusammen mit dem Peptid Arg-Gly-Asp-Ser.

9. Medikament mit mindestens einem Peptid nach Anspruch 3 oder 4 als aktivem Wirkstoff zusammmen mit einem Peptid der Formel Arg-Gly-Asp-Ser.

10. Verfahren zur Herstellung eines Medikaments zur Verhinderung und/oder Behandlung von Thrombosen, dadurch gekennzeichnet, daß es in einer pharmazeutisch verträglichen Form eingenommen werden kann, möglicherweise zusammen mit einer anderen Verbindung mit antithrombotischer Aktivität, einem Peptid der, wie in Anspruch 6 definierten, allgemeinen Formel I oder II.

## Claims

1. A peptide of the general formula Ia :

$$Z\text{-}Lys\text{-}Arg\text{-}Asp\text{-}X\text{-}Y \quad \text{(IA)}$$

wherein :
 – X represents the residue of one of the 20 common amino-acids in the L-configuration,
 – Y represents hydroxyl or the C-terminal residue of one of the 20 common amino-acids in the L-configuration,
 – Z represents acetyl, hydrogen or the N-terminal residue of one of the 20 common amino-acids in the L-configuration, possibly N-acetylated, with the provisos that :
 – when Y represents the C-terminal residue of one of the 20 common amino-acids in the L-configuration, Z represents acetyl or hydrogen,
 – X can in all cases be different from Y and from Z, and
 – when Y is hydroxyl and Z is hydrogen, X is not the C-terminal residue of valine.

2. A peptide according to claim 1, characterized in that it is of the general formula IIa :

$$Lys\text{-}Arg\text{-}Asp\text{-}X \quad \text{(IIA)}$$

wherein X is defined as in claim 1, with the proviso that X cannot represent the C-terminal residue of valine.

3. The peptide Lys-Arg-Asp-Ser.

4. A peptide selected from the group consisting of :

9